# EUROPÄISCHE PATENTSCHRIFT

(11) **EP 1 587 612 B1**
(45) Veröffentlichungstag und Bekanntmachung des Hinweises auf die Patenterteilung: **29.08.2018**
(21) Anmeldenummer: 03702582.2
(22) Anmeldetag: 31.01.2003
(51) Int. Cl.: B01J 8/06, B01J 19/00

(54) **MANTELROHRREAKTOR ZUR DURCHFÜHRUNG KATALYTISCHER GASPHASENREAKTIONEN UND VERFAHREN ZUM BETREIBEN EINES SOLCHEN**
TUBULAR REACTOR FOR CARRYING OUT CATALYTIC GAS-PHASE REACTIONS AND METHOD FOR OPERATING SAID REACTOR
REACTEUR TUBULAIRE A ENVELOPPE POUR EXECUTER DES REACTIONS CATALYTIQUES EN PHASE GAZEUSE ET PROCEDE POUR FAIRE FONCTIONNER UN TEL REACTEUR TUBULAIRE A ENVELOPPE

(43) Veröffentlichungstag der Anmeldung: 26.10.2005
(73) Patentinhaber: MAN Diesel & Turbo SE, 86153 Augsburg (DE)
(72) Erfinder: GÜTLHUBER, Friedrich, 85579 Neubiberg (DE); LEHR, Manfred, 94469 Deggendorf (DE)
(74) Vertreter: Paustian, Othmar
(86) Internationale Anmeldenummer: PCT/EP2003/000977
(87) Internationale Veröffentlichungsnummer: WO 2004/067164

(56) Entgegenhaltungen:
- WO-A-01/85332
- WO-A-03/022418
- DE-A- 19 807 018
- US-A1- 2003 017 095

## Beschreibung

Die Erfindung betrifft einen Mantelrohrreaktor gemäß Gattungsbegriff des Patentanspruchs 1 sowie Verwendungen und ein Verfahren zum Betreiben eines solchen Reaktors.

Ein derartiger Mantelrohrreaktor ist etwa aus WO 01/85332 A bekannt. In diesem besonderen Fall allerdings wird im Bestreben, ein Explosionsrisiko herabzusetzen, eine explosionskritische Komponente des in dem Reaktor zur Reaktion gebrachten Prozeßgases zum Teil erst unmittelbar vor oder in den Reaktionsrohren zugesetzt. Ferner ist das dieser Komponente bis dahin zur Verfügung stehende Volumen, etwa durch einen Einbau in eine ansonsten übliche, mehr oder weniger kalottenförmige Gaseintrittshaube, gering gehalten. Diese Maßnahmen beruhen auf folgenden Erkenntnissen:
1) Um im Verhältnis zur Größe der Reaktoranlage eine möglichst große Produktionsleistung zu erzielen, ist es wünschenswert, die Beladung des Prozeßgases mit den explosionskritischen Komponenten, wie z.B. Sauerstoff und Kohlenwasserstoff, möglichst groß machen zu können.
2) Außer mit der Beladung nimmt das Explosionsrisiko zu mit der Dauer, während welcher die beiden Komponenten beieinander weilen.

Vorausgehend hat man sich gegenüber umfangreicheren Schäden beim eventuellen Auftreten von Explosionen durch den Einbau von Berstscheiben in Reaktoranlagen zu schützen gesucht. Will man indessen die Beladung und damit die Ausbeute weiter steigern, so ist auf Grund des erhöhten Explosionsrisikos die Verwendung von Berstscheiben ungenügend. Der Ersatz der - an sich schon teueren - Berstscheiben im Explosionsfall erfordert verhältnismäßig langwierige Reparaturarbeiten und entsprechende Ausfallzeiten. Das Bersten von Berstscheiben ist verbunden mit einer nach außen gelangenden Druckwelle, die als Knall kilometerweit zu hören und schon deshalb vielfach untragbar ist. Dazu noch können schädliche Gase in die Umwelt austreten. Ferner ist das nach einer Explosion und dem dadurch notwendigen Ersatz von Berstscheiben jeweils erforderliche Wiederanfahren des Reaktors schwierig und zeitraubend, zumal zum Erreichen einer hohen Beladung im Betriebszustand beim Anfahren häufig darauf zu achten ist, daß das Durchschreiten eines Explosionsbereichs des in den Reaktor momentan eingespeisten Gasgemischs vermieden wird.

Ein solcher Explosionsbereich läßt sich in einem Zwei- oder Dreikomponentendiagramm darstellen, wie etwa in "Handbuch des Explosionsschutzes" von Henrikus Steen, Verlag WILEY-VCH, 1. Auflage (2000), Seite 332, angegeben, wobei die dritte Komponente ein zur Verdünnung zugesetztes Inertgas, wie zum Beispiel Stickstoff, ist. Es zeigt sich, daß Explosionsgefahr nur innerhalb eines darüber hinaus von Druck, Temperatur und Geometrie abhängigen fensterartigen Bereichs eines solchen Diagramms besteht.

Nach DE 198 06 810 A1 kann die Temperatur des gaseintrittsseitigen Rohrbodens durch eine darauf aufgebrachte Wärmeisolationsschicht herabgesetzt werden, um schädliche Nebenreaktionen einschließlich Zündungen und Deflagrationen zu unterbinden.

EP 1 180 508 A1 lehrt, beim Anfahren eines Reaktors den Explosionsbereich durch ständige Messung und Änderung der Prozeßgaszusammensetzung zu umgehen, wobei zunächst ein Inertgas zugesetzt wird, das nach Einsetzen der Reaktion mehr und mehr durch bereits reagiertes Prozeßgas ersetzt wird.

Auf dieser Grundlage liegt der vorliegenden Erfindung in erster Linie die Aufgabe zugrunde, auf risikolose und dazu noch ökonomische Weise die Beladung des zur Verarbeitung gelangenden Prozeßgases weiter steigern zu können.

Diese Aufgabe ist erfindungsgemäß gelöst mit den Merkmalen des Patentanspruchs 1, wozu diejenigen der Unteransprüche beitragen.

Als zweites liegt der Erfindung die Aufgabe zugrunde, einen erfindungsgemäßen Mantelrohrreaktor unter Ausnutzung seiner besonderen Eigenschaften wirtschaftlich zu betreiben. Diese Aufgabe ist gelöst durch einen jeden der Verwendungs- bzw. Verfahrensansprüche 32 bis 35.

Der erfindungsgemäße Reaktor vermag zum einen selbst mit explosionskritischer Beladung des zur Verarbeitung gelangenden Prozeßgases sicher betrieben zu werden, zum anderen beim Anfahren einen zündfähigen Bereich zu durchfahren, was den Anfahrprozeß erheblich erleichtert und beschleunigt.

Für die nachfolgenden Betrachtungen ist zu unterscheiden zwischen einer Explosion (englisch: deflagration) und einer Detonation (englisch: explosion oder auch detonation) - worauf allerdings in der vorgenannten, auf einer Übersetzung aus dem Japanischen beruhenden Literaturstelle EP 1 180 508 A1 nicht geachtet ist -). Im Gegensatz zu einer Explosion, die an einer Stelle einsetzt und eine sich mit Unterschallgeschwindigkeit fortpflanzende Druckwelle hervorruft, ist eine Detonation ein erheblich plötzlicherer und entsprechend heftigerer Vorgang, der neben einer zumeist noch spezielleren Gasmischung eine vorausgehende Explosion voraussetzt, die sich über eine bestimmte, konstruktionsabhängige Anlaufstrecke entwickeln kann.

Nachfolgend werden nun einige Ausführungsbeispiele der Erfindung anhand der begleitenden Zeichnungen genauer beschrieben. Von diesen letzteren zeigt:
Fig. 1 einen gaseintrittsseitigen Rohrboden samt Gaseintrittshaube eines erfindungsgemäßen Mantelrohrreaktors in einem halben Längsschnitt,
Fig. 2 einen Querschnitt durch den Randbereich des in Fig. 1 gezeigten Rohrbodens in Höhe der Linie II-II von Fig. 1,
Fig. 3 und Fig. 4 Details ähnlich denjenigen nach Fig. 1 und 2, jedoch bei einer Ausführungsform mit einem Einbau in eine konventionelle Gaseintrittshaube für die Zuführung des Prozeßgases,
Fig. 5 einen halben Längsschnitt ähnlich demjenigen von Fig. 1 durch den gaseintrittsseitigen Rohrboden, eine konventionelle, schalenförmige Gaseintrittshaube und einen darin vorgesehenen Einbau ähnlich demjenigen von Fig. 3,
Fig. 6a) bis Fig. 6f) jeweils eine Ausführungsform einer teildurchlässigen Dichtung, wie sie in Fig. 5 zu erkennen ist, in größerem Maßstab,
Fig. 7 eine ähnliche Darstellung wie Fig. 5, jedoch bei einer anderen Ausführungsform,
Fig. 8 ein Schema einer innerhalb eines erfindungsgemäßen Mantelrohrreaktors angeordneten Abstützung für vor allem den gaseintrittsseitigen Rohrboden,
Fig. 9 ein Schema einer Gaseintrittshaube ähnlich derjenigen von Fig. 5 mit daran vorgesehenen Kühl- und/oder Heizmitteln.
Fig. 10 eine ähnliche Darstellung wie Fig. 7 mit hinsichtlich des dem Reaktor zugeführten Prozeßgasstromes vorausgehenden erfindungsgemäßen Einrichtungen, und
Fig. 11 ein Schema einer alternativen erfindungsgemäßen Prozeßgaszuführung in Verbindung mit einer Gaseintrittshaube gemäß Fig. 1.

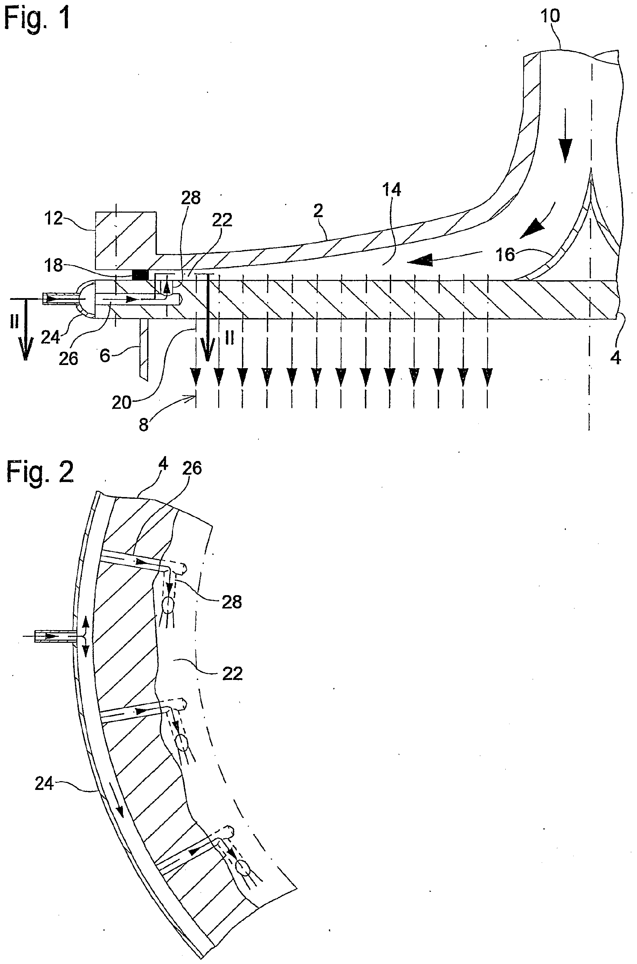

Fig. 1 zeigt etwas schematisch das Gaseintrittsende eines erfindungsgemäßen Mantelrohrreaktors für die Durchführung katalytischer Gasphasenreaktionen im explosions- oder gar detonationskritischen Bereich. Genauer gesagt sind in Fig. 1 eine besonders gestaltete Gaseintrittshaube 2, der darunterliegende Rohrboden 4, der daran anschließende Reaktormantel 6, ein hier nur gestrichelt angedeutetes ringförmiges Kontaktrohrbündel 8 und ein in die Gaseintrittshaube 2 mündender Gaseintrittsrohrstutzen 10 zu erkennen. In üblicher Weise ist das Rohrbündel 8, das eine geeignete Katalysatorfüllung enthält, innerhalb des Reaktormantels 6 von einem - im Betrieb jedenfalls - flüssigen Wärmeträger umspült, über den entlang den Kontaktrohren ein geeignetes Temperaturprofil aufrechterhalten und überschüssige Reaktionswärme abgeführt wird.

Wie aus Fig. 1 weiter erkennbar, ist die Gaseintrittshaube 2, abgesehen von einem massiven, ihrer Anbringung und Abdichtung an dem Rohrboden 4 dienenden peripheren Bund 12, verhältnismäßig flach und in etwa trompetenschallbecherartig ausgebildet, so daß sich zwischen ihr und dem Rohrboden 4 ein flacher Gasverteilungsraum 14 ergibt, der sich stetig, d.h. ohne Absätze, Knickstellen oder dergl., an den Gaseintrittsrohrstutzen 10 anschließt. Die Anbringung der Gaseintrittshaube 2 an dem Rohrboden 4 erfolgt über hier nur andeutungsweise dargestellte ringsherum, angeordnete Schraubbolzen.

Der Gasverteilungsraum 14 wird so dimensioniert, daß das über ihn den Kontaktrohren zugeführte Prozeßgas möglichst gleichförmig, d.h. insbesondere verwirbelungs- wie aber auch verweilfrei den Kontaktrohren zufließt. Dabei kann die Bemessung des Gasverteilungsraumes etwa eine solche sein, daß die Radialstromkomponente oder aber der statische Druck in dem Prozeßgas in Radialrichtung konstant bleibt. Auch Mischformen sind denkbar wie andererseits die trompetenschallbecherartige Form der Gaseintrittshaube 2 auch durch mehr oder weniger konische Ringelemente (nicht gezeigt) angenähert sein kann. Zur Herstellung der Stetigkeit des Gasstromes am Eintritt in den Gasverteilungsraum 14 ist dort, unterhalb des Gaseintrittsrohrstutzens 10 und auf dem Rohrboden 4 aufsitzend, ein dornenförmiger Strömungsleitkörper 16 angeordnet, der zugleich einen Verdränger bildet, um das Gas an einem frontalen Auftreffen auf der Mitte des Rohrbodens 4 zu hindern. Die Minimalhöhe des Gasverteilungsraumes 14 wird in dem gezeigten Beispiel von einem Dichtungsring 18 definierter Stärke bestimmt, mit dem der Gasverteilungsraum 14 nach außen zu abgedichtet ist. Sie wird im Zuge der Planung des Reaktors festgelegt und muß jedenfalls so groß sein, daß sie an keiner Stelle des Reaktorumfangs, etwa auf Grund von Unebenheiten an Haube 2 und/oder Rohrboden 4, zu null wird. Erforderlichenfalls müssen Haube und/oder Rohrboden an der nämlichen Stelle planbearbeitet werden.

Da sich indessen radial außerhalb der äußersten Kontaktrohre, wie zum Beispiel 20, ein Totvolumen 22 innerhalb des Gasverteilungsraumes 14 konstruktiv kaum vermeiden läßt, ohne den Gaseintritt in die äußersten Kontaktrohre zu behindern, und ein solches Totvolumen zu einem ungewollten Verweilen des Prozeßgases führen würde, sind an dieser Stelle Maßnahmen getroffen, um das Prozeßgas aus dem Totvolumen 22 zu verdrängen oder zumindest auf eine explosionsunkritische Zusammensetzung zu "verdünnen". Dies geschieht durch Einblasen eines in bezug auf die zu befürchtende Explosionsreaktion inaktivierenden Gases. Ein solches kann ein Inertgas sein, wie etwa N₂, ein im Verlauf der betriebsmäßig durchgeführten Reaktion anfallendes Beiprodukt, wie etwa CO₂, zuweilen einfach Luft oder auch ein Gemisch solcher Gase.

Gemäß Fig. 1 wird das betreffende Gas - nachfolgend Spülgas genannt - über eine Ringleitung 24 an der Peripherie des Rohrbodens 4, davon nach innen zu abzweigende Stichkanäle 26 in regelmäßigen Abständen entlang dem Umfang des Rohrbodens 4 und von den Stichkanälen 26 nach oben abzweigende Düsenbohrungen 28 eingeblasen.

Wie in Fig. 2 zu erkennen, sind die Düsenbohrungen 28 in Umfangsrichtung des Rohrbodens 4 geneigt, um dem daraus austretenden Gas eine radiale Strömungskomponente zu vermitteln und auf diese Weise das gesamte Totvolumen 22 zu durchspülen.

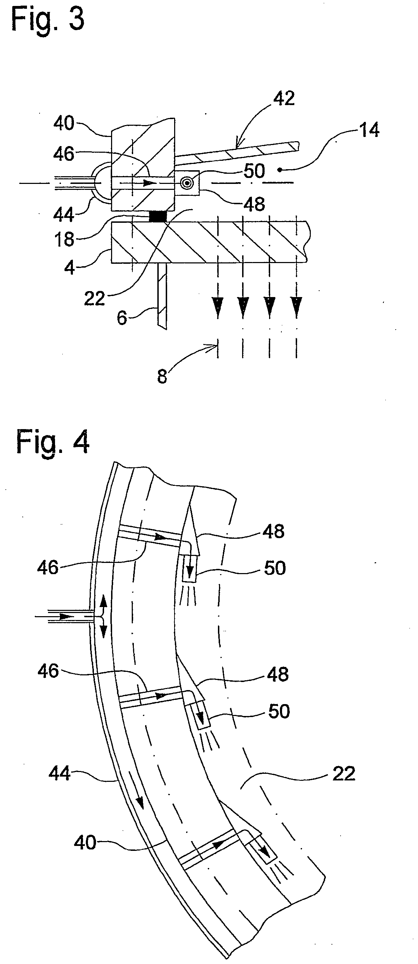

Die Figuren 3 und 4 zeigen eine andere Ausführungsform des Gaseintrittsendes eines Mantelrohrreaktors nach der Erfindung. Hier ist innerhalb einer insoweit üblichen schalenförmigen Gaseintrittshaube, von der nur der Rand 40 dargestellt ist, ein den Gasverteilungsraum 14 begrenzender Einbau 42 zu erkennen. - Soweit die hier wie im folgenden dargestellten Teile mit denen nach Fig. 1 und 2 identisch sind, tragen sie die gleichen Bezugszahlen.

In weiterer Abweichung von dem vorausgehend beschriebenen Ausführungsbeispiel umgibt hier eine Ringleitung 44 für in das Totvolumen 22 einzuführendes Spülgas den Rand 40 der Gaseintrittshaube, und entsprechend sind Stichkanäle 46 vergleichbar den Stichkanälen 26 radial durch den Rand 40 hindurchgeführt. Die Stichkanäle 46 münden in innenseitig an dem Rand 40 angebrachten Düsenaufsätzen 48 mit tangential gerichteten Düsen 50 für den Gasaustritt, wiederum, um das Totvolumen 22 möglichst vollständig zu spülen.

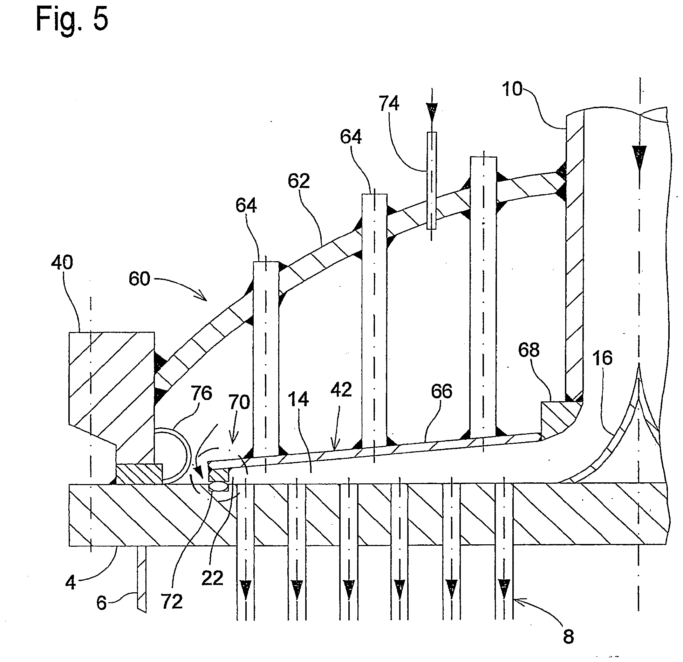

Fig. 5 zeigt eine Anordnung insoweit ähnlich derjenigen nach Fig. 3 als auch hier ein Einbau 42 innerhalb einer schalenförmigen Gaseintrittshaube vorgesehen ist. Wie ersichtlich, ist der Einbau 42 frei hängend an der Gaseintrittshaube 60, genauer gesagt deren Schale 62, mittels Stehbolzen 64 wie auch des Gaseintrittsrohrstutzens 10 verankert in einer Weise, daß ggf. in dem Gasverteilungsraum 14 auftretende Explosions- oder gar Detonationskräfte in die Schale 62 eingeleitet werden. Um diese Kräfte bestmöglich aufnehmen zu können, ist die Schale 62 kalottenförmig.

In diesem Ausführungsbeispiel ist der Einbau 42 aus einer schwach konischen Ringscheibe 66 und einem nach innen und unten abgerundeten Profilring 68 zusammengesetzt und an seinem Rand 70 über eine teildurchlässige Dichtung 72 auf dem Rohrboden 4 abgestützt, während die Gaseintrittshaube 60 außerhalb des Einbaus 42 von dem Spülgas für das Totvolumen 22 erfüllt ist. Von dort tritt das Spülgas in einem Maße, wie es der Gaseintrittshaube 60 laufend über eine Leitung 74 zugeführt wird, über die teildurchlässige Dichtung 72 ringsherum gleichmäßig in das Totvolumen 22 ein.

Die Gaseintrittshaube 60, genauer gesagt deren massiver Rand 40, ist gegenüber dem Rohrboden 4 in diesem Beispiel über eine Art Schweißlippendichtung 76 abgedichtet, ähnlich wie in DE 44 07 728 C1 beschrieben. Indessen könnte auch hier wieder ein Dichtungsring wie der Dichtungsring 18 aus den vorausgehend beschriebenen Ausführungsbeispielen Verwendung finden. Zweckmäßigerweise steht das Spülgas gegenüber der Außenatmosphäre wie selbstredend auch gegenüber dem Gasverteilungsraum 14 unter Überdruck, um als Sperrmedium zu fungieren.

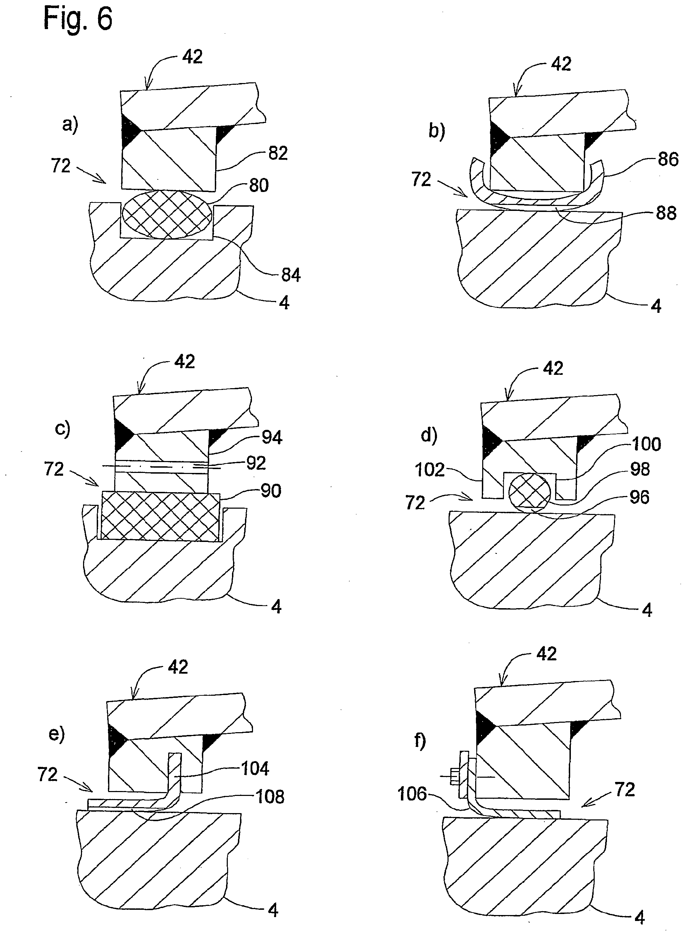

Die Figuren 6a) bis 6f) zeigen verschiedene gegenwärtig in Betracht gezogene Ausführungsformen für die teildurchlässige Dichtung 72 aus Fig. 5. Nach Fig. 6a) besteht die teildurchlässige Dichtung 72 aus einem Ring 80 von Haus aus kreisrunden oder auch bereits elliptischen Querschnitts aus einem porösen, schwach kompressiblen Material, wie zum Beispiel Graphitgewebe, der von einem ringförmigen Ansatz 82 des Einbaus 42 in eine entsprechende Ringnut 84 des Rohrbodens 4 gepreßt wird. Nach Fig. 6b) besteht die Dichtung 72 aus einem C-förmig profilierten, vorzugsweise metallenen Reifen 86, der auf seiner Außenseite, zum Rohrboden 4 hin, eine Vielzahl regelmäßig verteilter radialer oder auch etwas tangentialer Riefen 88 aufweist, nach Fig. 6c) aus einem massiven elastischen Dichtungsring 90 ähnlich etwa dem Dichtungsring 18 in Verbindung mit radialen oder auch etwas tangentialen Bohrungen 92 in einem Ansatz 94 ähnlich dem Ansatz 82 aus Fig. 6a). Nach Fig. 6d) wird die Dichtung 72 von einem mit radialen oder etwas tangentialen Riefen 96 versehenen Ring 98 im wesentlichen kreisförmigen Querschnitts aus Metall oder einem anderen hartelastischen Material gebildet, der in einer Ringnut 100 innerhalb eines Ansatzes 102 ähnlich dem Ansatz 82 liegt. Nach den Figuren 6e) und 6f) finden Blechprofilringe 104 bzw. 106 abgewinkelten Querschnitts als Dichtung 72 Verwendung, die wiederum, wie aus Fig. 6e) ersichtlich, zum Rohrboden 4 hin Riefen, 108, ähnlich den Riefen 88 aufweisen können. Solche Profilringe können gegenüber einem einseitig wirksamen Überdruck nachgiebig sein, um so einen mehr oder weniger großen Durchtrittsquerschnitt für das Spülgas freizugeben.

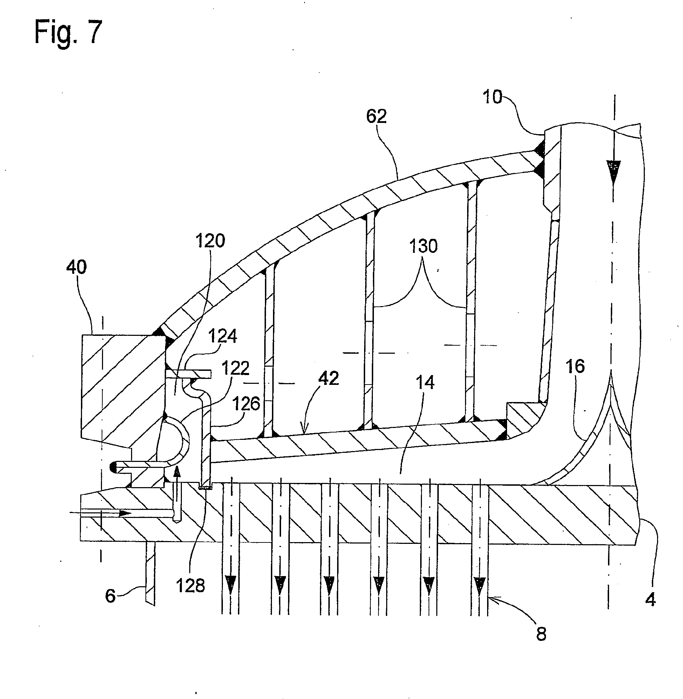

Fig. 7 zeigt eine Anordnung ähnlich derjenigen aus Fig. 5, bei der jedoch das Spülgas durch den Rohrboden 4 hindurch in einen ringförmigen Raum 120 zwischen einer Schweißlippendichtung 122 ähnlich der Schweißlippendichtung 76 und zwei dicht aneinanderschließenden Blechringen 124 und 126 radial außerhalb des Einbaus 42 eintritt. Der im wesentlichen zylindrische Blechring 126, der dicht am Rand des Einbaus 42 angebracht ist, reicht lose in eine Ringnut 128 des Rohrbodens 4 hinein, um so eine teildurchlässige Dichtung zum Gasverteilungsraum 14 hin zu bilden, ähnlich der teildurchlässigen Dichtung 72 aus Fig. 5. Die Stehbolzen 64 aus Fig. 5 sind in diesem Beispiel durch durchbrochene zylindrische Bleche 130 ersetzt.

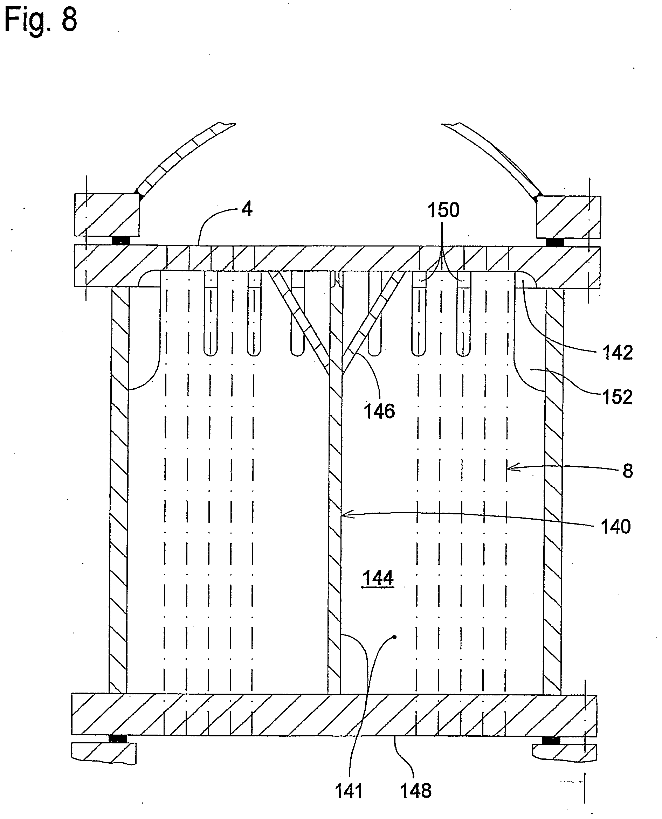

Fig. 8 zeigt wie der gaseintrittsseitige Rohrboden 4 für den Fall, daß im Gaseintrittsbereich doch einmal eine Explosion oder gar Detonation auftritt, zum Gasaustrittsende des Reaktors hin abgestützt sein kann. Im gezeigten Beispiel besteht eine entsprechende Abstützung 140 aus einem mehrflügeligen Metallbauteil, im wesentlichen gebildet von zwei stehend kreuzweise angeordneten Blechen 141, die - vorzugsweise lose - in entsprechende Nuten 142 auf der Unterseite des Rohrbodens 4 eingreifen und in entsprechenden radialen Gassen des Rohrbündels 8 Platz finden. Zusätzlich kann das Zentrum des Rohrbodens 4, wie gezeigt, innerhalb des rohrfreien Mittelbereichs 144 nahe der Berohrung durch schräge Streben oder einen Blechkegel 146 an den Blechen 141 abgestützt sein. Dies erlaubt es unter Umständen mit nur einem Blech 141 auszukommen, womit sich dann u.a. auch zwei rohrfreie Gassen einsparen lassen. An die Stelle des Blechkegels 146 könnte auch ein zylindrisches, prismatisches oder pyramidenförmiges Metallbauteil treten.

Die Abstützung 140 kann, muß jedoch nicht, wie gezeigt, bis zum gasaustrittsseitigen Rohrboden 148 - oder einer Trennscheibe - durchlaufen. Auf jeden Fall aber muß sie in der Lage sein, die Abstützkräfte in den Reaktormantel 6 einzuleiten. Zum Ausgleich unterschiedlicher Wärmedehnungen können Bleche die Bleche wie 141, vor allem in der Nähe des gaseintrittsseitigen Rohrbodens 4, längsgerichtete Spannungsentlastungsschlitze 150 sowie an ihrem Anschluß an den Reaktormantel 6 entsprechende Aussparungen 152 aufweisen. Im übrigen können sie, wo immer dies aus strömungstechnischen Gesichtspunkten oder wegen Gewichtseinsparung zweckmäßig sein mag, durchbrochen oder durch eine Gerüstkonstruktion ersetzt sein. Ein Aufsitzen der Abstützung 140 auf dem gasaustrittsseitigen Rohrboden 148 hat nicht zuletzt den Vorteil, daß dann auch dieser letztere gegenüber Explosionsdruckkräften abgestützt ist, die sich durch die Berohrung hindurch dem Gasaustrittsraum mitteilen oder dort durch Nachzündung entstehen können.

Gemäß DE 198 06 810 A1 kann der gaseintrittsseitige Rohrboden 4 wärmeisoliert sein (nicht gezeigt), um den Gasverteilungsraum 14 "kühl" zu halten und auch damit eine Explosions- oder gar Detonationsneigung herabzusetzen.

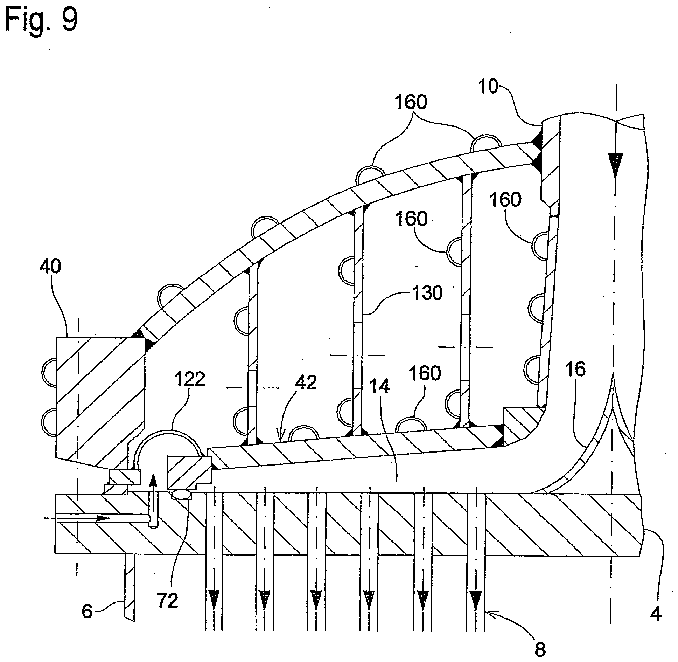

Fig. 9 sieht zum gleichen Zweck - bei einer Anordnung ähnlich derjenigen aus Fig. 5 oder 7 - an diversen Stellen in und an der Gaseintrittshaube 2 Kühlmittelkanäle 160 vor, die jedoch auch, insbesondere beim Anfahren des Reaktors, als Heizmittelkanäle dienen und im übrigen dazu beitragen können, Wärmespannungen abzubauen.

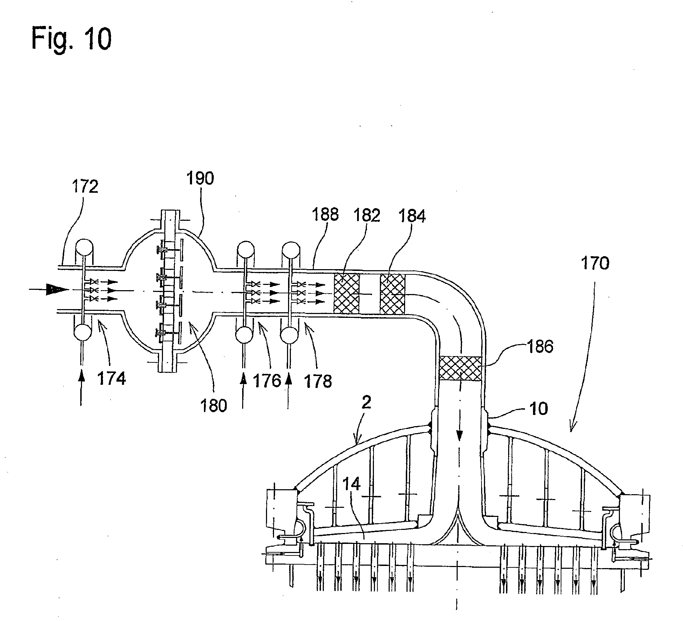

Fig. 10 zeigt das Gaseintrittsende 170 eines Mantelrohrreaktors gemäß Fig. 7 mit vorausgehenden Mitteln zur Aufbereitung des Prozeßgases. In dem gezeigten Beispiel wird in eine Hauptzuleitung 172 für eine geeignet temperierte und unter geeignetem Druck stehende Prozeßgasgrundkomponente wie z.B. Luft, den sogenannten Hauptstrom, an einer beliebigen Stelle 174 eine zweite Prozeßgaskomponente, wie z.B. ein Kohlenwasserstoffgas, in einer Menge eingespeist, die noch kein explosionsfähiges Gemisch ergibt, während weitere Teilmengen der zweiten oder auch weitere Prozeßgaskomponenten bei 176 und 178 im Anschluß an eine Rückschlagventilanordnung 180 zugesetzt werden. Spätestens nach der Einspeisungsstelle 178 befindet sich das Prozeßgas im explosiblen Bereich.

Die gesamten zugeführten Prozeßgaskomponenten werden anschließend mittels mehrerer aufeinanderfolgender Mischer 182, 184 und 186 schonend, d.h. beispielsweise unter weitgehender Vermeidung von Turbulenzen, in mehreren Koordinaten vermischt. Dabei wird zudem bei der Leitungsführung auf die Vermeidung von Unstetigkeiten geachtet. Auch ist die Leitung 188 zwischen Rückschlagventilanordnung 180 und Gaseintrittshaube 2 so kurz wie möglich gehalten, um der Entstehung hoher Explosionsdrücke vorzubeugen. Das gilt vor allem dann, wenn ein Detonationsanlauf zu befürchten ist. Die Rückschlagventilanordnung 180 verhindert, daß eine ggf. in der Leitung 188 oder anschließend entstehende Druckwelle sich in die Zuleitung 172 hinein fortsetzen und in den diese speisenden Organen Schaden anrichten kann. Die Rückschlagventilanordnung 180 befindet sich in einer Kammer 190, die zugleich ein erwünschtes Druckentspannungsvolumen für eine solche Druckwelle bildet. Die Kammer 190 kann eine weitgehend beliebige Gestalt und ein nach oben hin weitgehend beliebiges Volumen besitzen, ebenso wie auch weitere Kammern an der nämlichen Stelle hinzutreten können. Gewünschtenfalls kann im übrigen auch auf die erste Einspeisungsstelle, 174, zweckmäßigerweise noch vor der Rückschlagventilanordnung 180, bereits ein Mischer folgen (nicht gezeigt). Indessen kann die Einspeisungsstelle 174 weit vor der Rückschlagventilanordnung 180 liegen, um bereits auf diese Weise eine gute Durchmischung zu erreichen. Andererseits kann ggf. im Anschluß an die Rückschlagventilanordnung 180 eine einzige zusätzliche Einspeisungsstelle wie z.B. 176 und ein einziger Mischer genügen.

Es versteht sich, daß die Rückschlagventilanordnung 180, die Kammer 190, die Leitung 188 samt darin enthaltenen Mischern 182 - 186 und Einspeisungsorganen wie auch der Reaktor selbst festigkeitsmäßig dafür ausgelegt sein müssen, dem schlimmstenfalls darin bzw. daran auftretenden Explosions- bzw. Detonationsdruck standzuhalten. Dies gilt, wie gesagt, trotz der vorausgehend beschriebenen Maßnahmen, um Detonationen wie auch bereits Explosionen möglichst zu vermeiden.

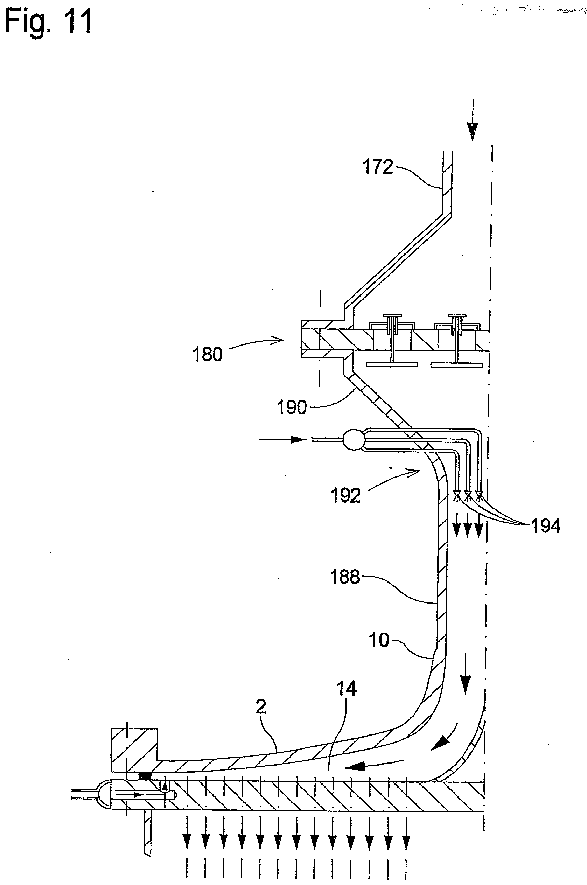

Fig. 11 zeigt eine Anordnung prinzipiell ähnlich derjenigen nach Fig. 10, allerdings in Verbindung mit einer Gaseintrittshaube 2 gemäß Fig. 1 und unter Weglassung von Mischern sowie einer Krümmung in der Leitung 188, die in diesem Fall besonders kurz ausfällt. Mischer führen in jedem Fall zu Störungen der Gasströmung, womit das betreffende Prozeßgas explosionsanfälliger wird. Für die Herstellung besonders explosionskritischer Prozeßgasgemische sucht man Mischer deshalb möglichst zu vermeiden. Ferner sucht man die für die Entwicklung einer Detonation in Betracht kommende Anlaufstrecke zu verkürzen.

Nach Fig. 11 nun ist die Rückschlagventilanordnung 180 zentral über der Gaseintrittshaube 2 auf der Achse des Gaseintrittsrohrstutzens 10 angeordnet und anstelle der beiden Einspeisungsstellen 176 und 178 aus Fig. 10 eine einzige Feineindüsungsstelle bzw. -vorrichtung 192 vorgesehen, während Mischer fehlen. Die Feineindüsungsvorrichtung 192 weist eine Vielzahl, d.h. mindestens 5, vorzugsweise jedoch 50 pro m² oder noch mehr, über den Leitungsquerschnitt verteilter Eindüsungsorgane 194 auf, die mit Düsen und individuellen Drosselorganen versehen ähnlich den Eindüsungsorganen am Kontaktrohreintritt nach DE 100 21 986 A1 ausgebildet sein können und/oder der eingedüsten Prozeßgaskomponente einen Drall zu vermitteln vermögen. Auf diese Weise läßt sich die Einspeisung der zweiten Prozeßgaskomponente, wie z.B. eines Kohlenwasserstoffs, so fein verteilt und regelmäßig vornehmen, daß sich Mischer für die Herstellung eines homogenen Prozeßgasstromes erübrigen.

Prinzipiell kann die eingedüste Prozeßgaskomponente in flüssiger wie in gasförmiger Form, kalt oder aufgeheizt, vorliegen. Bei flüssiger Form ist es denkbar, sie mittels eines Inertgases einzublasen. So oder so kann die Eindüsung mit hohem Druck erfolgen, um eine Teilverdampfung, verbunden mit einem Strahlaufreißen, herbeizuführen, ähnlich wie es auch bei der Kraftstoffzuführung zum Zylinderraum von Verbrennungsmotoren praktiziert wird.

Der Eindüsungsbereich kann mit einer Mantelheizung ausgestattet sein, und entsprechend können die Zuführungsrohre für die zweite Prozeßkomponente beheizt oder wärmeisoliert sein.

Die Festigkeitsbemessung der Reaktorkomponenten hängt von Art und Konzentration der zu verarbeitenden Stoffe ab. Sie wird gewöhnlich für den stationären Betriebsfall vorgenommen. Beim Anfahren eines Mantelrohrreaktors der vorausgehend beschriebenen Art achtet man folglich darauf, daß zu keiner Zeit die für den Betrieb veranschlagte Explosionsstärke überschritten wird. Man startet in der Regel mit nur einer, d.h. der jeweiligen Prozeßgasgrundkomponente (Hauptstrom) . Ist ein bestimmter Massenstrom hiervon erreicht, so gibt man die zweite Prozeßgaskomponente hinzu. Wird im Betrieb von der Anlage selbst ein Inertgas, wie z.B. CO₂, erzeugt, so kann das Anfahren unter dessen Heranziehung im wesentlichen nach EP 1 180 508 A1 erfolgen. Ob beim Anfahren zusätzlich ein Inertgas zuzuführen ist oder ob die Explosions- bzw. Detonationsheftigkeit etwa allein durch Variation von Druck und Temperatur auf den Betriebsfall reduziert werden kann, richtet sich nach der Verfahrensauslegung.

Wie eingangs bereits erwähnt, kann und darf das Anfahren bereits in den zündfähigen Bereich führen. Wie im Betrieb sind auch beim Anfahren neben der Prozeßgaszusammensetzung weitere Parameter, so vor allem Druck und Temperatur, zu berücksichtigen. Beide nehmen Einfluß auf das Explosions- wie auch das Detonationsverhalten. Denkbar ist, während des Anfahrens Druck und Temperatur zu variieren. So kann etwa beim Anfahren der Druck reduziert werden, während die Temperatur im Gasverteilungsraum 14 angehoben wird. Spätestens gegen Ende der Anfahrphase werden beide dann auf die vorgesehenen Betriebswerte gebracht.

Wird ein Mantelrohrreaktor im unteren Explosionsbereich, d.h. mit nur geringem Explosionsrisiko und geringem in Rechnung zu stellendem Explosionsdruck, betrieben und wird dabei dem Hauptstrom ein Recyclegas aus dem Reaktor als Inertgas zugesetzt, so kann das Anfahren beispielsweise folgendermaßen erfolgen:
Zunächst wird über die Hauptzuleitung 172 als Hauptstrom Luft bzw. Sauerstoff zugeführt. Dann beginnt man, etwa über die Eindüsungsvorrichtung 194 (Fig. 11), einen Kohlenwasserstoffstrom hinzuzugeben. Solange die Kohlenwasserstoffkonzentration gering ist, ist ein Explosionsrisiko nicht gegeben. Ebenso besteht das gewonnene Recyclegas im wesentlichen nur aus dem Stoff des Hauptstroms. Bei fortgeschrittenem Anfahrprozeß unter Zusatz von Kohlenwasserstoff wird bereits Reaktionsprodukt erzeugt, wodurch das Recyclegas schon einen Anteil Inertgas wie z.B. Kohlendioxid enthält. Im weiteren Verlauf des Anfahrprozesses wird der Kohlenwasserstoffstrom verstärkt. Da der Hauptstrom dann aber bereits einen signifikanten Anteil des Inertgases enthält, wird zu keiner Zeit ein kritischer Zustand erreicht. Grundsätzlich versucht man auf solche Weise beim Anfahren den explosiblen Bereich zu vermeiden, um erst bei Erreichen genügender Prozeßstabilität in den Explosionsbereich einzutreten.

Prinzipiell gleiches gilt auch für einen Betrieb im oberen Explosionsbereich. Hier allerdings wird in der Regel über die Zuleitung 172 der Kohlenwasserstoffstrom als Hauptstrom zugeführt, während etwa über die Eindüsungsvorrichtung 194 Sauerstoff eingespeist wird.

Nach derzeitigem Kenntnisstand kann ein erfindungsgemäßer Mantelrohrreaktor mit Vorteil für Oxidations-, Hydrierungs-, Dehydrierungs-, Nitrierungs-, Alkylierungsprozesse und dergl. Verwendung finden und dabei vor allem für die Herstellung von Ketonen, Methylisobutylketon, Mercaptan, Isopren, Anthrachinon, o-Kresol, Ethylenhexan, Furfurol, Acetylen, Vinylacetat, Isopropylchlorid, Naphtalsäureanhydrid, Vinylchlorid, Oxoalkohol, Pyrotol, Styrol, Methansäurenitril, Polyphenylenoxid, Dimethylphenol, Pyridinaldehyd, Therban, Alphaolefinen, Vitamin B6, Blausäure, Anilin, Methansäurenitral, Difluormethan, 4-Methyl-2-Pentanon und Tetrahydrofuran sowie im besonderen die
Oxidation von Dimethylbenzolen (m,o,p) zu den entsprechenden Mono- und Dialdehyden,
Oxidation von Dimethylbenzolen (m,o,p) zu den entsprechenden Mono- und Dicarbonsäuren bzw. deren Anhydriden,
Oxidation von Trimethylbenzolen zu den entsprechenden Mono-, Di- und Trialdehyden,
Oxidation von Trimethylbenzolen zu den entsprechenden Mono-, Di- und Tricarbonsäuren bzw. deren Anhydriden,
Oxidation von Durol zu Pyromellithsäureanhydrid,
Oxidation von gamma- bzw. beta-Picolin zu gamma- bzw. beta-Picolincarbaldehyd,
Oxidation von gamma- bzw. beta-Picolin zu Isonicotinsäure bzw. Nicotinsäure,
Oxidation von Propen zu Acrolein,
Oxidation von Acrolein zu Acrylsäüre,
Oxidation von Propan zu Acrolein,
Oxidation von Propan zu Acrylsäure,
Oxidation von Butan zu MSA,
Oxidation von Raffinat zu MSA,
Oxidation von i-Buten zu Methacrolein,
Oxidation von Methacrolein zu Methacrylsäure,
Oxidation von Methacrolein zu Methylmethacrylat,
Oxidation von i-Butan zu Methacrolein,
Oxidation von i-Butan zu Methacrylsäure,
Ammonoxidation von Dimethylbenzolen (m,o,p) zu den entsprechenden Mono- und Dinitrilen,
Ammonoxidation von Trimethylbenzolen zu den entsprechenden Mono- und Di- bzw. Trinitrilen,
Ammonoxidation von Propan zu Acrylnitril,
Ammonoxidation von Propen zu Acrylnitril,
Ammonoxidation von beta-Picolin zu 3-Cyanopyridin, Ammonoxidation von gamma-Picolin zu 4-Cyanopyridin,
Oxidation von Methanol zu Formaldehyd,
Oxidation von Naphthalin und/oder O-Xylol, ggf. im Mischbetrieb, zu Phthalsäureanhydrid,
Oxidation von Ethan zu Essigsäure,
Oxidation von Ethanol zu Essigsäure,
Oxidation von Geraniol zu Citral,
Oxidation von Ethen zu Ethylenoxid,
Oxidation von Propen zu Propylenoxid,
Oxidation von Chlorwasserstoff zu Chlor,
Oxidation von Glykol zu Glyoxal und
Hydrierung von MSA zu Butandiol.

## Patentansprüche

1. Mantelrohrreaktor zur Durchführung katalytischer Gasphasenreaktionen und mit einem von dem betreffenden Reaktionsgasgemisch durchströmten, eine Katalysatorfüllung aufweisenden, sich zwischen einem gaseintrittsseitigen Rohrboden (4) und einem gasaustrittsseitigen Rohrböden (148) erstreckenden und innerhalb eines umgebenden Reaktormantels (6) von einem Wärmeträger umspülten Kontaktrohrbündel (8) sowie die beiden Rohrböden überspannenden Gaseintritts- bzw. Gasaustrittshauben (2; 60) für die Zuführung des betreffenden Prozeßgases zu dem bzw. die Abführung des reagierten Prozeßgases von den Kontaktrohren und mit einer Prozeßgas-Zuleitung (172) zum Einleiten des Prozeßgases in die Gaseintrittshaube (2), **dadurch gekennzeichnet, daß**
die Prozeßgas-Zuleitung (172) einen ersten Abschnitt zur Aufnahme eines nicht explosiblen Prozeßgases und in Strömungsrichtung des Prozeßgases dahinter einen zweiten Abschnitt mit einer Einspeisungsstelle (176, 178; 192) zur Aufnahme eines explosiblen Prozeßgases aufweist;
die Prozeßgas-Zuleitung (172) in dem ersten Abschnitt eine Rückschlagventilanordnung (180) aufweist,
ein Druckentspannungsvolumen in dem ersten Abschnitt in Strömungsrichtung nach der Rückschlagventilanordnung angeordnet ist, wobei das Druckentspannungsvolumen zumindest teilweise von einer die Rückschlagventilanordnung (180) aufnehmenden Kammer (190) gebildet wird;
und
die Rückschlagventilanordnung (180) und der gaseintrittsseitige Rohrboden (4) sowie sämtliche zwischen diesen befindliche, den Prozeßgasdruck tragenden Teile für den maximalen Druck, der bei der Explosion oder Detonation des Prozeßgases auftritt, festigkeitsmäßig ausgelegt sind.

2. Mantelrohrreaktor nach Anspruch 1, **gekennzeichnet durch** eine Einrichtung zum Einblasen eines in bezug auf die betreffende Reaktion inerten Spülgases in Toträume, in denen das Prozeßgas vor Eintritt in die Kontaktrohre ansonsten ganz oder teilweise zur Ruhe kommen würde.

3. Mantelrohrreaktor nach Anspruch 2, **gekennzeichnet durch** eine Einrichtung zum Einblasen von Spülgas am Rand des gaseintrittsseitigen Rohrbodens (4) radial außerhalb des Kontaktrohrbündels (8).

4. Mantelrohrreaktor nach Anspruch 3, **gekennzeichnet durch** eine Einrichtung zum Einblasen des betreffenden Spülgases mit einer tangentialen Strömungskomponente.

5. Mantelrohrreaktor nach einem der vorhergehenden Ansprüche, **dadurch gekennzeichnet, daß** die Gaseintrittshaube (2) flach trichterförmig mit radial nach außen kleiner werdendem Abstand zum gaseintrittsseitigen Rohrboden (4) und mit zentralem Gaseintritt ausgebildet ist.

6. Mantelrohrreaktor nach Anspruch 5, **dadurch gekennzeichnet, daß** die Gaseintrittshaube (2) zumindest annähernd trompetenschallbecherartig abgerundet und sich zum Rand hin abflachend ausgebildet ist.

7. Mantelrohrreaktor nach einem der Ansprüche 1 bis 4, **dadurch gekennzeichnet, daß** in einer insoweit herkömmlichen, schalenförmigen Gaseintrittshaube (60) koaxial ein flacher trichterförmiger Einbau (42) angeordnet ist, von dem eine zentrale Durchbrechung abgedichtet mit dem Gaseintritt in Verbindung steht und dessen Rand zum Rand des gaseintrittsseitigen Rohrbodens (4) hin abgedichtet ist.

8. Mantelrohrreaktor nach Anspruch 7, **dadurch gekennzeichnet, daß** der Einbau (42) zumindest annähernd trompetenschallbecherartig abgerundet und sich zum Rand hin abflachend ausgebildet ist.

9. Mantelrohrreaktor nach Anspruch 7 oder 8, **dadurch gekennzeichnet, daß** der Einbau (42) an mehreren, vorzugsweise regelmäßig verteilten Stellen an der Gaseintrittshaube (60) abgestützt ist.

10. Mantelrohrreaktor nach einem der Ansprüche 7 bis 9 in Verbindung mit Anspruch 4, **dadurch gekennzeichnet, daß** die Abdichtung (72) am Rand des Einbaus (42) beschränkt gasdurchlässig ist und das betreffende Spülgas über sie eingeblasen wird.

11. Mantelrohrreaktor nach Anspruch 10, **dadurch gekennzeichnet, daß** die betreffende Abdichtung (72) aus einem teildurchlässigen Material wie z.B. Graphitgewebe besteht.

12. Mantelrohrreaktor nach Anspruch 10, **dadurch gekennzeichnet, daß** die betreffende Abdichtung (72) diskrete Gasdurchtrittskanäle wie z.B. Bohrungen (92) oder Riefen (88; 96; 108) aufweist.

13. Mantelrohrreaktor nach Anspruch 10 oder 11, **dadurch gekennzeichnet, daß** die betreffende Abdichtung (72) aus einem ggf. gegen Überdruck nachgiebigen Profil (86; 104; 106) besteht.

14. Mantelrohrreaktor nach einem der Ansprüche 10 bis 13, **dadurch gekennzeichnet, daß** die betreffende Abdichtung (72) außenseitig mit einem Raum in Verbindung steht, durch den hindurch das Spülgas zugeführt wird.

15. Mantelrohrreaktor nach Anspruch 14, **dadurch gekennzeichnet, daß** der betreffende Raum von einer radial inneren Dichtung (72) und einer radial äußeren Dichtung (76) begrenzt ist.

16. Mantelrohrreaktor nach Anspruch 15, **gekennzeichnet durch** eine Einrichtung, mit der das Spülgas gegenüber der Außenatmosphäre unter Überdruck setzbar ist.

17. Mantelrohrreaktor nach einem der Ansprüche 14 bis 16, **dadurch gekennzeichnet, daß** der betreffende Raum im wesentlichen aus dem Restvolumen der Gaseintrittshaube (60) besteht.

18. Mantelrohrreaktor nach einem der vorhergehenden Ansprüche, **dadurch gekennzeichnet, daß** die Gaseintrittshaube (2; 60), der gaseintrittsseitige Rohrboden (4) und/oder, soweit vorhanden, der betreffende Einbau (42) untereinander über eine Schweißlippendichtung (76; 122) in Verbindung stehen.

19. Mantelrohrreaktor nach einem der vorhergehenden Ansprüche, **dadurch gekennzeichnet, daß** auf dem gaseintrittsseitigen Rohrboden (4), zum Gaseintritt hin gerichtet, ein sich nach dorthin verjüngender, dornförmiger Strömungsleitkörper (16) angeordnet ist.

20. Mantelrohrreaktor nach einem der vorhergehenden Ansprüche, **dadurch gekennzeichnet, daß** zwischen dem gaseintrittsseitigen Rohrboden (4) und dem gasaustrittsseitigen Rohrboden (148) eine Abstützung für den gaseintrittsseitigen Rohrboden (4) am Reaktormantel (6) befestigt ist.

21. Mantelrohrreaktor nach Anspruch 20, **dadurch gekennzeichnet, daß** die Abstützung zumindest teilweise aus einem in bezug auf die Reaktorlängsmittelachse mehrflügeligen Metallbauteil besteht.

22. Mantelrohrreaktor nach Anspruch 21 und mit ringförmigem Kontaktrohrbündel (8), **dadurch gekennzeichnet, daß** die Abstützung teilweise aus einem zusätzlichen im wesentlichen zylindrischen, prismatischen, konischen oder pyramidenförmigen Metallbauteil im rohrfreien Innenraum des Kontaktrohrbündels besteht, das sich seinerseits an dem mehrflügeligen Metallbauteil abstützt.

23. Mantelrohrreaktor nach einem der vorhergehenden Ansprüche, **dadurch gekennzeichnet, daß** der gaseintrittsseitige Rohrboden (4) wärmeisoliert ist.

24. Mantelrohrreaktor nach einem der vorhergehenden Ansprüche, **dadurch gekennzeichnet, daß** in der Prozeßgas-Zuleitung (172) eine erste Prozeßgaskomponente strömt und die Prozeßgas-Zuleitung (172) in Strömungsrichtung des Prozeßgases vor der Rückschlagventilanordnung (180) eine erste Einspeisungsstelle (174) für eine zweite Prozeßgaskomponente, die der ersten Prozeßgaskomponente zugesetzt wird, aufweist.

25. Mantelrohrreaktor nach Anspruch 24, **dadurch gekennzeichnet, daß** auf zumindest die letzte Einspeisungsstelle (178) mindestens ein Mischer (182, 184, 186) folgt.

26. Mantelrohrreaktor nach Anspruch 24 oder 25, **dadurch gekennzeichnet, daß** zumindest eine zweite Einspeisungsstelle von einer Feineindüsungsvorrichtung (192) mit einer Mehrzahl über den Kanalquerschnitt verteilten Eindüsungsorganen (194) gebildet wird.

27. Mantelrohrreaktor nach Anspruch 26, **dadurch gekennzeichnet, daß** die Eindüsungsorgane (194) mit individuellen Drosselorganen und/oder einen Drall vermittelnden Organen ausgestattet sind.

28. Mantelrohrreaktor nach einem der Ansprüche 24 bis 27, **dadurch gekennzeichnet, daß** zumindest eine der Einspeisungsstellen (174, 176, 178; 192) dazu ausgelegt ist, die betreffende Prozeßgaskomponente in flüssiger Form, ggf. aufgeheizt, aufzunehmen oder selbst aufzuheizen.

29. Mantelrohrreaktor nach Anspruch 28, **dadurch gekennzeichnet, daß** die betreffende Einspeisungsstelle (174, 176, 178; 192) Mittel zum Einblasen der flüssigen Prozeßgaskomponente aufweist.

30. Mantelrohrreaktor nach Anspruch 28 oder 29, **dadurch gekennzeichnet, daß** die betreffende Einspeisungsstelle (174, 176, 178; 192) in der Lage ist, die betreffende Prozeßgaskomponente zu zerstäuben und/oder zu verdampfen.

31. Mantelrohrreaktor nach einem der Ansprüche 24 bis 30, **dadurch gekennzeichnet, daß** die Einspeisungsstelle (174, 176, 178; 192) und/oder ihre Zuführung Heizmittel aufweist und/oder wärmeisoliert ist.

32. Verwendung eines Mantelrohrreaktors nach einem der vorhergehenden Ansprüche für Oxidations-, Hydrierungs-, Dehydrierungs-, Nitrierungs-, Alkylierungsprozesse und dergleichen.

33. Verwendung nach Anspruch 32, **dadurch gekennzeichnet, daß** der Mantelrohrreaktor für die Herstellung von Ketonen, Methylisobutylketon, Mercaptan, Isopren, Anthrachinon, o-Kresol, Ethylenhexan, Furfurol, Acetylen, Vinylacetat, Isopropylchlorid, Naphtalsäureanhydrid, Vinylchlorid, Oxoalkohol, Pyrotol, Styrol, Methansäurenitril, Polyphenylenoxid, Dimethylphenol , Pyridinaldehyd, Therban, Alphaolefinen, Vitamin B6, Blausäure, Anilin, Methansäurenitral, Difluormethan, 4-Methyl-2-Pentanon und Tetrahydrofuran sowie im besonderen die
Oxidation von Dimethylbenzolen (m,o,p) zu den entsprechenden Mono- und Dialdehyden,
Oxidation von Dimethylbenzolen (m,o,p) zu den entsprechenden Mono- und Dicarbonsäuren bzw. deren Anhydriden, Oxidation von Trimethylbenzolen zu den entsprechenden Mono-, Di- und Trialdehyden,
Oxidation von Trimethylbenzolen zu den entsprechenden Mono-, Di- und Tricarbonsäuren bzw. deren Anhydriden,
Oxidation von Durol zu Pyromellithsäureanhydrid,
Oxidation von gamma- bzw. beta-Picolin zu gamma- bzw. beta-Picolincarbaldehyd,
Oxidation von gamma- bzw. beta-Picolin zu Isonicotinsaure bzw. Nicotinsäure,
Oxidation von Propen zu Acrolein,
Oxidation von Acrolein zu Acrylsäure,
Oxidation von Propan zu Acrolein,
Oxidation von Propan zu Acrylsäure,
Oxidation von Butan zu MSA,
Oxidation von Raffinat zu MSA,
Oxidation von i-Buten zu Methacrolein,
Oxidation von Methacrolein zu Methacrylsäure,
Oxidation von Methacrolein zu Methylmethacrylat,
Oxidation von i-Butan zu Methacrolein,
Oxidation von i-Butan zu Methacrylsäure,
Ammonoxidation von Dimethylbenzolen (m,o,p) zu den entsprechenden Mono- und Dinitrilen,
Ammonoxidation von Trimethylbenzolen zu den entsprechenden Mono- und Di- bzw. Trinitrilen,
Ammonoxidation von Propan zu Acrylnitril,
Ammonoxidation von Propen zu Acrylnitril,
Ammonoxidation von beta-Picolin zu 3-Cyanopyridin,
Ammonoxidation von gamma-Picolin zu 4-Cyanopyridin,
Oxidation von Methanol zu Formaldehyd,
Oxidation von Naphthalin und/oder O-Xylol, ggf. im Mischbetrieb, zu Phthalsäureanhydrid,
Oxidation von Ethan zu Essigsäure,
Oxidation von Ethanol zu Essigsäure, Oxidation von Geraniol zu Citral,
Oxidation von Ethen zu Ethylenoxid,
Oxidation von Propen zu Propylenoxid,
Oxidation von Chlorwasserstoff zu Chlor,
Oxidation von Glykol zu Glyoxal und
Hydrierung von MSA zu Butandiol,
Verwendung findet.

34. Verfahren zum Betreiben eines Mantelrohrreaktors nach einem der Ansprüche 1 bis 31, **dadurch gekennzeichnet, daß** der Mantelrohrreaktor im Produktionsbetrieb mit einer solchen Beladung einer ersten Prozeßgaskomponente mit mindestens einer weiteren Prozeßgaskomponente betrieben wird, bei der gelegentliche Explosionen oder gar Detonationen in Kauf genommen werden.

35. Verfahren zum Betreiben eines Mantelrohrreaktors nach einem der Ansprüche 1 bis 31, **dadurch gekennzeichnet, daß** zum Anfahren des Reaktors die Konzentrationen der Prozeßgaskomponenten und ggf. weitere Parameter laufend so bemessen werden, daß die Heftigkeit sich ggf. einstellender Explosionen oder gar Detonationen diejenige für den Betriebszustand in Kauf genommener Explosionen bzw. Detonationen nicht überschreitet.

## Claims

1. A shell-and-tube type reactor for carrying out catalytic gas phase reactions and comprising a contact tube bundle (8) through which the respective reaction gas mixture flows and which includes a catalyst filling, extends between a gas-inlet-side tubesheet (4) and a gas-outlet-side tubesheet (148) and is flushed with a heat carrier within a surrounding reactor shell (6) and comprising gas inlet and gas outlet hoods (2; 60) spanning the two tubesheets for supplying the respective process gas to the contact tubes and for discharging the reacted process gas from the contact tubes and comprising a process gas feed conduit (172) for introducing the process gas into the gas inlet hood (2), **characterized in that**
the process gas feed conduit (172) includes a first portion for taking up a non-explosive process gas and behind the same in the flow direction of the process gas a second portion with a feed point (176, 178; 192) for taking up an explosive process gas;
the process gas feed conduit (172) in the first portion includes a check valve assembly (180),
a depressurizing volume in the first portion is arranged downstream of the check valve assembly in the direction of flow, wherein the depressurizing volume is at least partly formed by a chamber (190) accommodating the check valve assembly (180); and
the check valve assembly (180) and the gas-inlet-side tubesheet (4) as well as all parts present between the same, which bear the process gas pressure, have a strength designed for the maximum pressure that occurs during the explosion or detonation of the process gas.

2. The shell-and-tube type reactor according to claim 1, **characterized by** a device for injecting a purge gas inert with respect to the respective reaction into dead spaces in which the process gas otherwise would wholly or partly come to rest before entry into the contact tubes.

3. The shell-and-tube type reactor according to claim 2, **characterized by** a device for injecting purge gas at the edge of the gas-inlet-side tubesheet (4) radially outside the contact tube bundle (8).

4. The shell-and-tube type reactor according to claim 3, **characterized by** a device for injecting the respective purge gas with a tangential flow component.

5. The shell-and-tube type reactor according to any of the preceding claims, **characterized in that** the gas inlet hood (2) has the shape of a flat funnel with a distance to the gas-inlet-side tubesheet (4) decreasing radially to the outside and with a central gas inlet.

6. The shell-and-tube type reactor according to claim 5, **characterized in that** the gas inlet hood (2) is at least approximately rounded like a trumpet bell and flattens towards the edge.

7. The shell-and-tube type reactor according to any of claims 1 to 4, **characterized in that** in a cup-shaped gas inlet hood (60), which in so far is conventional, a flat funnel-shaped fitting (42) is coaxially arranged, a central aperture of which is in sealed connection with the gas inlet and whose edge is sealed towards the edge of the gas-inlet-side tubesheet (4).

8. The shell-and-tube type reactor according to claim 7, **characterized in that** the fitting (42) is at least approximately rounded like a trumpet bell and flattens towards the edge.

9. The shell-and-tube type reactor according to claim 7 or 8, **characterized in that** the fitting (42) is supported on the gas inlet hood (60) at a plurality of preferably regularly distributed points.

10. The shell-and-tube type reactor according to any of claims 7 to 9 in conjunction with claim 4, **characterized in that** the seal (72) at the edge of the fitting (42) is gas-permeable to a limited extent and the respective purge gas is injected via the same.

11. The shell-and-tube type reactor according to claim 10, **characterized in that** the respective seal (72) is made of a partly permeable material such as e.g. graphite fabric.

12. The shell-and-tube type reactor according to claim 10, **characterized in that** the respective seal (72) includes discrete gas through-flow passages such as e.g. bores (92) or flutes (88; 96; 108).

13. The shell-and-tube type reactor according to claim 10 or 11, **characterized in that** the respective seal (72) is made of a profile (86; 104; 106) which is resilient to overpressure, if required.

14. The shell-and-tube type reactor according to any of claims 10 to 13, **characterized in that** on its outside the respective seal (72) is connected with a space through which the purge gas is supplied.

15. The shell-and-tube type reactor according to claim 14, **characterized in that** the respective space is defined by a radially inner seal (72) and a radially outer seal (76).

16. The shell-and-tube type reactor according to claim 15, **characterized by** a device by means of which the purge gas can be put under an overpressure with respect to the outside atmosphere.

17. The shell-and-tube type reactor according to any of claims 14 to 16, **characterized in that** the respective space substantially consists of the residual volume of the gas inlet hood (60).

18. The shell-and-tube type reactor according to any of the preceding claims, **characterized in that** the gas inlet hood (2; 60), the gas-inlet-side tubesheet (4) and/or, as far as present, the respective fitting (42) are connected to each other via a weld lip seal (76; 122).

19. The shell-and-tube type reactor according to any of the preceding claims, **characterized in that** on the gas-inlet-side tubesheet (4), directed towards the gas inlet, a mandrel-like flow guiding body (16) tapering thereto is arranged.

20. The shell-and-tube type reactor according to any of the preceding claims, **characterized in that** between the gas-inlet-side tubesheet (4) and the gas-outlet-side tubesheet (148) a support for the gas-inlet-side tubesheet (4) is attached to the reactor shell (6).

21. The shell-and-tube type reactor according to claim 20, **characterized in that** the support at least partly consists of a multi-blade metal component with respect to the reactor longitudinal central axis.

22. The shell-and-tube type reactor according to claim 21 and comprising a ring-shaped contact tube bundle (8), **characterized in that** the support partly consists of an additional substantially cylindrical, prismatic, conical or pyramid-shaped metal component in the tube-free interior space of the contact tube bundle, which in turn is supported on the multi-blade metal component.

23. The shell-and-tube type reactor according to any of the preceding claims, **characterized in that** the gas-inlet-side tubesheet (4) is thermally insulated.

24. The shell-and-tube type reactor according to any of the preceding claims, **characterized in that** a first process gas component flows in the process gas feed conduit (172) and upstream of the check valve assembly (180) in the flow direction of the process gas the process gas feed conduit (172) includes a first feed point (174) for a second process gas component that is added to the first process gas component.

25. The shell-and-tube type reactor according to claim 24, **characterized in that** at least the last feed point (178) is followed by at least one mixer (182, 184, 186).

26. The shell-and-tube type reactor according to claim 24 or 25, **characterized in that** at least one second feed point is formed by a fine injection device (192) with a plurality of injection members (194) distributed over the channel cross-section.

27. The shell-and-tube type reactor according to claim 26, **characterized in that** the injection members (194) are equipped with individual throttle members and/or members imparting a spin.

28. The shell-and-tube type reactor according to any of claims 24 to 27, **characterized in that** at least one of the feed points (174, 176, 178; 192) is designed to take up the respective process gas component in liquid form, possibly heated, or to heat it up itself.

29. The shell-and-tube type reactor according to claim 28, **characterized in that** the respective feed point (174, 176, 178; 192) includes means for injecting the liquid process gas component.

30. The shell-and-tube type reactor according to claim 28 or 29, **characterized in that** the respective feed point (174, 176, 178; 192) is able to atomize and/or vaporize the respective process gas component.

31. The shell-and-tube type reactor according to any of claims 24 to 30, **characterized in that** the feed point (174, 176, 178; 192) and/or its supply includes heating means and/or is thermally insulated.

32. Use of a shell-and-tube type reactor according to any of the preceding claims for oxidation, hydrogenation, dehydrogenation, nitration, alkylation processes and the like.

33. The use according to claim 32, **characterized in that** the shell-and-tube type reactor is employed for the production of ketones, methyl isobutyl ketone, mercaptan, isoprene, anthraquinone, o-cresol, ethylene hexane, furfurol, acetylene, vinyl acetate, isopropyl chloride, naphthalic anhydride, vinyl chloride, oxo alcohol, pyrotol, styrene, methanoic nitrile, polyphenylene oxide, dimethyl phenol, pyridine aldehyde, Therban, alpha-olefins, vitamin B6, hydrocyanic acid, aniline, methanoic nitral, difluoromethane, 4-methyl-2-pentanone and tetrahydrofurane, and in particular the
oxidation of dimethylbenzenes (m,o,p) to the corresponding mono- and dialdehydes,
oxidation of dimethylbenzenes (m,o,p) to the corresponding mono- and dicarboxylic acids and their anhydrides,
oxidation of trimethylbenzenes to the corresponding mono-, di- and trialdehydes, oxidation of trimethylbenzenes to the corresponding mono-, di- and tricarboxylic acids and their anhydrides,
oxidation of durene to pyromellitic anhydride,
oxidation of gamma- or beta-picoline to gamma- or beta-picoline carbaldehyde,
oxidation of gamma- or beta-picoline to isonicotinic acid or nicotinic acid,
oxidation of propene to acrolein,
oxidation of acrolein to acrylic acid,
oxidation of propane to acrolein,
oxidation of propane to acrylic acid,
oxidation of butane to MSA,
oxidation of raffinate to MSA,
oxidation of i-butene to methacrolein,
oxdiation of methacrolein to methacrylic acid,
oxdiation of methacrolein to methyl methacrylate,
oxidation of i-butane to methacrolein,
oxidation of i-butane to methacrylic acid,
ammoxidation of dimethylbenzenes (m,o,p) to the corresponding mono- and dinitriles,
ammoxidation of trimethylbenzenes to the corresponding mono- and di- or trinitriles,
ammoxidation of propane to acrylonitrile,
ammoxidation of propene to acrylonitrile,
ammoxidation of beta-picoline to 3-cyanopyridine,
ammoxidation of gamma-picoline to 4-cyanopyridine,
oxidation of methanol to formaldeyde,
oxidation of naphthalene and/or o-xylene, if required in a mixing operation, to phthalic anhydride,
oxidation of ethane to acetic acid,
oxidation of ethanol to acetic acid,
oxidation of geraniol to citral,
oxidation of ethene to ethylene oxide,
oxidation of propene to propylene oxide,
oxidation of hydrogen chloride to chlorine,
oxidation of glycol to glyoxal, and
hydrogenation of MSA to butanediol.

34. A method for operating a shell-and-tube type reactor according to any of claims 1 to 31, **characterized in that** in the production mode the shell-and-tube type reactor is operated with such a loading of a first process gas component together with at least one further process gas component that occasional explosions or even detonations of such a loading are accepted.

35. A method for operating a shell-and-tube type reactor according to any of claims 1 to 31, **characterized in that** for starting the reactor the concentrations of the process gas components and possibly further parameters continuously are dimensioned such that the intensity of possibly occurring explosions or even detonations does not exceed the intensity of explosions or detonations accepted for the operating condition.

## Revendications

1. Réacteur tubulaire à enveloppe pour l'exécution de réactions catalytiques en phase gazeuse et comprenant un faisceau de tubes de contact (8) traversés par l'écoulement du mélange de gaz de réaction concerné, qui comporte un remplissage catalyseur, qui s'étend entre un fond à tubes (4) du côté de l'entrée des gaz et un fond à tubes (148) du côté de la sortie des gaz, et entouré par l'écoulement d'un fluide caloporteur à l'intérieur d'une enveloppe de réacteur (6) qui l'entoure, et comprenant des coiffes d'entrée de gaz et de sortie de gaz (2 ; 60) qui couvrent les deux fonds à tubes pour l'admission du gaz de process concerné vers les tubes de contact et pour l'évacuation des gaz de process ayant réagi hors des tubes de contact, et comprenant un conduit d'amenée pour gaz de process (172) destiné à l'introduction du gaz de process dans la coiffe d'entrée de gaz (2),
**caractérisé en ce que**
le conduit d'amenée pour gaz de process (172) comporte une première portion pour recevoir un gaz de process non explosif et, derrière celui-ci dans la direction d'écoulement du gaz de process, une seconde portion avec un emplacement d'alimentation (176, 178 ; 192) pour recevoir un gaz de process explosif ;
le conduit d'amenée pour gaz de process (172) comporte dans la première portion un agencement à clapet antiretour (180),
un volume de détente de pression est agencé dans la première portion après l'agencement à clapet antiretour dans la direction d'écoulement, ledit volume de détente de pression étant au moins partiellement formé par une chambre (190) qui reçoit l'agencement à clapet antiretour (180) ; et l'agencement à clapet antiretour (180) et le fond à tube (4) du côté de l'entrée des gaz ainsi que la totalité des pièces situées entre ceux-ci et encaissant la pression du gaz de process sont conçus en termes de solidité pour la pression maximale qui se produit lors de l'explosion ou de la détonation du gaz de process.

2. Réacteur tubulaire à enveloppe selon la revendication 1, **caractérisé par** un système pour insuffler un gaz de rinçage inerte à l'égard de la réaction concernée dans les volumes morts dans lesquels le gaz de process viendrait, avant l'entrée dans les tubes de contact, sinon entièrement ou partiellement en situation de repos.

3. Réacteur tubulaire à enveloppe selon la revendication 2, **caractérisé par** un système pour insuffler un gaz de rinçage au niveau de la bordure du fond à tube (4) du côté de la sortie de gaz et radialement à l'extérieur du faisceau de tubes de contact (8).

4. Réacteur tubulaire à enveloppe selon la revendication 3, **caractérisé par** un système pour insuffler le gaz de rinçage concerné avec une composante d'écoulement tangentielle.

5. Réacteur tubulaire à enveloppe selon l'une des revendications précédentes, **caractérisé en ce que** la coiffe d'entrée de gaz (2) est réalisée plate et en forme d'entonnoir avec une distance par rapport au fond à tube (4) du côté de l'entrée de gaz qui diminue radialement vers l'extérieur et avec une entrée de gaz au centre.

6. Réacteur tubulaire à enveloppe selon la revendication 5, **caractérisé en ce que** la coiffe d'entrée de gaz (2) est arrondie au moins approximativement sous la forme d'un pavillon de trompette et est réalisée de manière à s'aplatir en direction du bord.

7. Réacteur tubulaire à enveloppe selon l'une des revendications 1 à 4, **caractérisé en ce que**, dans une coiffe d'entrée de gaz (60) par ailleurs classique et en forme de coque, est agencé coaxialement un insert plat en forme d'entonnoir (42) lequel communique avec l'entrée de gaz via une traversée centrale et de façon étanche, et dont la bordure est étanchée vis-à-vis de la bordure du fond à tubes (4) du côté de l'entrée du gaz.

8. Réacteur tubulaire à enveloppe selon la revendication 7, **caractérisé en ce que** l'insert (42) est arrondi au moins approximativement sous la forme d'un pavillon de trompette et est réalisé de manière à s'aplatir en direction du bord.

9. Réacteur tubulaire à enveloppe selon la revendication 7 ou 8, **caractérisé en ce que** l'insert (42) est soutenu en plusieurs emplacements répartis, de préférence de façon régulière, sur la coiffe d'entrée de gaz (60).

10. Réacteur tubulaire à enveloppe selon l'une des revendications 7 à 9, en dépendance de la revendication 4, **caractérisé en ce que** l'étanchement (72) est perméable aux gaz de façon limitée à la bordure de l'insert (42), et le gaz de rinçage concerné est insufflé via celui-ci.

11. Réacteur tubulaire à enveloppe selon la revendication 10, **caractérisé en ce que** l'étanchement concerné (72) est en un matériau partiellement perméable, comme par exemple un textile à base de graphite.

12. Réacteur tubulaire à enveloppe selon la revendication 10, **caractérisé en ce que** l'étanchement concerné (72) comporte des canaux de sortie de gaz discret, comme des perçages (92) ou des stries (88 ; 96 ; 108).

13. Réacteur tubulaire à enveloppe selon la revendication 10 ou 11, **caractérisé en ce que** l'étanchement concerné (72) est constitué par un profilé (86 ; 104 ; 106) capable de céder le cas échéant sous une surpression.

14. Réacteur tubulaire à enveloppe selon l'une des revendications 10 à 13, **caractérisé en ce que** l'étanchement concerné (72) communique du côté extérieur avec un volume, le gaz de rinçage étant alimenté en traversant ledit volume.

15. Réacteur tubulaire à enveloppe selon la revendication 14, **caractérisé en ce que** le volume concerné est délimité par un joint radial intérieur (72) et par un joint radial extérieur (76).

16. Réacteur tubulaire à enveloppe selon la revendication 15, **caractérisé par** un système au moyen duquel le gaz de rinçage est susceptible d'être amené en surpression par rapport à l'atmosphère extérieure.

17. Réacteur tubulaire à enveloppe selon l'une des revendications 14 à 16, **caractérisé en ce que** le volume concerné est essentiellement constitué par le volume résiduel de la coiffe d'entrée de gaz (60).

18. Réacteur tubulaire à enveloppe selon l'une des revendications précédentes, **caractérisé en ce que** la coiffe d'entrée de gaz (2 ; 60) ; le fond à tubes (4) du côté de l'entrée des gaz et/ou, s'il est présent, l'insert concerné (42) sont en liaison les uns avec les autres au moyen d'un joint à lèvre soudé (76, 122).

19. Réacteur tubulaire à enveloppe selon l'une des revendications précédentes, **caractérisé en ce qu'**un corps de guidage d'écoulement (16) de forme pointue et en rétrécissement en direction de l'entrée des gaz est agencé sur le fond à tube (4) du côté de l'entrée du gaz et orienté vers l'entrée du gaz.

20. Réacteur tubulaire à enveloppe selon l'une des revendications précédentes, **caractérisé en ce que**, entre le fond à tubes (4) du côté de l'entrée des gaz et le fond à tubes (148) du côté de la sortie des gaz, un support est fixé pour soutenir le fond à tubes (4) du côté de l'entrée de gaz, sur l'enveloppe du réacteur (6).

21. Réacteur tubulaire à enveloppe selon la revendication 20, **caractérisé en ce que** le soutien est au moins partiellement constitué par un composant en métal à ailettes multiples par rapport à l'axe médian longitudinal du réacteur.

22. Réacteur tubulaire à enveloppe selon la revendication 21, comprenant un faisceau de tubes de contact (8) de forme annulaire, **caractérisé en ce que** le soutien est constitué partiellement par un composant en métal additionnel de forme sensiblement cylindrique, prismatique, conique ou pyramidale dans l'espace intérieur exempt de tubes du faisceau de tubes de contact, qui s'appuie à son tour sur le composant métallique à ailettes multiples.

23. Réacteur tubulaire à enveloppe selon l'une des revendications précédentes, **caractérisé en ce que** le fond à tubes (4) du côté de l'entrée du gaz est thermiquement isolé.

24. Réacteur tubulaire à enveloppe selon l'une des revendications précédentes, **caractérisé en ce qu'**un premier composant du gaz de process s'écoule dans le conduit d'amenée de gaz de process (172), et le conduit d'amenée de gaz de process (172) comporte, avant l'agencement à clapet antiretour (180) en direction d'écoulement du gaz de process, un premier emplacement d'injection (174) pour un second composant du gaz de process, qui est additionné au premier composant de gaz de process.

25. Réacteur tubulaire à enveloppe selon la revendication 24, **caractérisé en ce que**, au moins le dernier remplacement d'injection (178) est suivi par au moins un mélangeur (182, 184, 186).

26. Réacteur tubulaire à enveloppe selon la revendication 24 ou 25, **caractérisé en ce qu'**au moins un second emplacement d'injection est formé par un dispositif à buse fine (192) comportant une pluralité d'organes à buses (194) répartis sur la section du canal.

27. Réacteur tubulaire à enveloppe selon la revendication 26, **caractérisé en ce que** les organes à buses (194) sont équipés d'organes d'étranglement individuels et/ou d'organes imposant un tourbillonnement.

28. Réacteur tubulaire à enveloppe selon l'une des revendications 24 à 27, **caractérisé en ce que** l'un au moins des emplacements d'injection (174, 176, 178 ; 192) est conçu pour recevoir le composant concerné du gaz de process sous forme liquide, le cas échéant chauffé, ou encore pour le chauffer lui-même.

29. Réacteur tubulaire à enveloppe selon la revendication 28, **caractérisé en ce que** l'emplacement d'injection concerné (174, 176, 178 ; 192) comprend un moyen pour insuffler le composant liquide du gaz de process.

30. Réacteur tubulaire à enveloppe selon la revendication 28 ou 29, **caractérisé en ce que** l'emplacement d'injection concerné (174, 176, 178 ; 192) est en mesure de pulvériser et/ou de vaporiser le composant concerné du gaz de process.

31. Réacteur tubulaire à enveloppe selon l'une des revendications 24 à 30, **caractérisé en ce que** l'emplacement d'injection (174, 176, 178 ; 192) et/ou son admission comprend un moyen de chauffage et/ou est thermiquement isolé.

32. Utilisation d'un réacteur tubulaire à enveloppe selon l'une des revendications précédentes, pour des processus d'oxydation, d'hydratation, de déshydratation, de nitration, d'alkylation ou similaire.

33. Utilisation selon la revendication 32, **caractérisée en ce que** le réacteur tubulaire à enveloppe est utilisé pour la production de cétones, méthylisobutylcétone, mercaptan, isoprène, anthraquinone, o-crésol, éthylène hexane, furfurol, acétylène, acétate de vinyle, chlorure d'isopropyle, anhydride naphtalique, chlorure de vinyle, oxoalcool, pyrotole, styrène, méthanesonitrile, polyphénylène oxyde, diméthylphénol, pyridine aldéhyde, therban, alphaoléfines, vitamine B6, acide cyanhydrique, aniline, nitrure d'acide formique, difluorométhane, 4-méthyl-2-pentanone et tétrahydrofurane et en particulier
oxydation de diméthylbenzènes (m,o,p) en mono- et dialdéhydes correspondants,
oxydation de diméthylbenzènes (m,o,p) en acides mono- et dicarboxyliques correspondants ou leurs anhydrides,
oxydation de triméthylbenzènes en mono-, di- et trialdéhydes,
oxydation de triméthylbenzènes en acides mono-, di- et tricarboxyliques correspondants ou leurs anhydrides,
oxydation de durol en anhydride d'acide pyromellitique,
oxydation de gamma- ou de bêta-picoline en gamma- ou bêta-picoline carbaldéhyde,
oxydation de gamma- ou de bêta-picoline en acide isonicotinique ou en acide nicotinique,
oxydation de propène en acroléine,
oxydation d'acroléine en acide acrylique,
oxydation de propane en acroléine,
oxydation de propane en acide acrylique,
oxydation de butane en MSA,
oxydation de raffinat en MSA,
oxydation de i-butène en méthacroléine,
oxydation de méthacroléine en acide méthacrylique,
oxydation de méthacroléine en méthacrylate de méthyle,
oxydation de i-butane en méthacroléine,
oxydation de i-butane en acide méthacrylique,
ammoxydation de diméthylbenzènes (m,o,p) en mono- et dinitriles correspondants,
ammoxydation de triméthylbenzènes en mono- et di- ou trinitriles correspondants,
ammoxydation de propane en acrylonitrile,
ammoxydation de propène en acrylonitrile,
ammoxydation de beta-picoline en 3-cyanopyridine,
ammoxydation de gamma-picoline en 4-cyanopyridine,
oxydation de méthanol en formaldéhyde,
oxydation de naphtalène et/ou d'o-xylène, si nécessaire en mélange, en anhydride d'acide phtalique,
oxydation d'éthane en acide acétique,
oxydation d'éthanol en acide acétique,
oxydation de géraniol en citral,
oxydation d'éthène en oxyde d'éthylène,
oxydation de propène en oxyde de propylène,
oxydation du chlorure d'hydrogène en chlore,
oxydation de glycol en glyoxal et
hydrogénation de MSA en butanediol.

34. Procédé pour l'exploitation d'un réacteur tubulaire à enveloppe selon l'une des revendications 1 à 31,
**caractérisé en ce que** le réacteur tubulaire à enveloppe est amené à fonctionner en service de production avec un chargement d'un premier composant de gaz de process avec un autre composant de gaz de process pour lequel on s'attend à des explosions occasionnelles voire même à des détonations.

35. Procédé pour l'exploitation d'un réacteur tubulaire à enveloppe selon l'une des revendications 1 à 31,
**caractérisé en ce que** pour le démarrage du réacteur les concentrations des composants de gaz de process et le cas échéant d'autres paramètres sont réglé(e)s en continu de telle façon que la violence des explosions ou encore des détonations qui se produisent éventuellement ne dépasse pas celle des explosions ou des détonations prises en considération pour l'état de service.
